# EUROPÄISCHE PATENTSCHRIFT

(11) **EP 4 633 544 B1**
(45) Veröffentlichungstag und Bekanntmachung des Hinweises auf die Patenterteilung: **04.02.2026**
(21) Anmeldenummer: 23828704.9
(22) Anmeldetag: 11.12.2023
(51) Int. Cl.: A61F 2/64, A61F 2/66, A61F 2/74, A61F 2/50

(54) **ORTHOPÄDIETECHNISCHE GELENKEINRICHTUNG**
ORTHOPEDIC JOINT DEVICE
DISPOSITIF D'ARTICULATION ORTHOPÉDIQUE

(30) Priorität: 16.12.2022 DE 102022133654
(43) Veröffentlichungstag der Anmeldung: 22.10.2025
(73) Patentinhaber: Ottobock SE & Co. KGaA, 37115 Duderstadt (DE)
(72) Erfinder: NÖRTHEMANN, Jens, 37115 Duderstadt (DE); PUSCH, Martin, 37115 Duderstadt (DE); SCHUH, Andreas, 37115 Duderstadt (DE)
(74) Vertreter: Gramm, Lins & Partner Patent- und Rechtsanwälte PartGmbB
(86) Internationale Anmeldenummer: PCT/EP2023/085208
(87) Internationale Veröffentlichungsnummer: WO 2024/126420

(56) Entgegenhaltungen:
- DE-B4- 102017 124 337
- DE-U1- 29 723 632
- US-A1- 2008 255 670

## Beschreibung

Die Erfindung betrifft eine orthopädietechnische Gelenkeinrichtung mit einem Oberteil und einem Unterteil, die schwenkbar um eine gemeinsame Gelenkachse aneinander gelagert sind, und einer ein Hydraulikfluid aufweisende Rotationshydraulik, die ein Gehäuse mit einer Kammer und einem darin schwenkbar gelagerten Schwenkkolben aufweist, der die Kammer in eine Flexionskammer und eine Extensionskammer unterteilt, die über zumindest einen Kanal hydraulisch miteinander verbunden sind, wobei der Schwenkkolben eine Aufnahme aufweist, in oder an der eine in Richtung auf das Gehäuse wirkende Dichtung angeordnet ist.

Orthopädietechnische Gelenkeinrichtungen sind in Orthesen, Exoskeletten oder Exoprothesen angeordnet und dienen dazu, ein Oberteil gelenkig mit einem Unterteil zu verbinden. Zwischen dem Oberteil und dem Unterteil wird eine Gelenkachse ausgebildet, die bei einem monozentrischen Gelenk eine feste Zuordnung zu sowohl dem Oberteil als auch zu dem Unterteil hat. Bei einem polyzentrischen Gelenk kann die Gelenkachse sich relativ zu dem Oberteil oder dem Unterteil über den Verschwenkwinkel verändern. Zur Beeinflussung der Verschwenkbewegung von Oberteil und Unterteil ist an der orthopädietechnischen Gelenkeinrichtung ein Antrieb oder ein Dämpfer angeordnet. Der Dämpfer kann unterschiedlich ausgebildet sein und wandelt die Bewegungsenergie in eine andere Energieform um, insbesondere in Wärmeenergie. Häufig sind die Widerstandseinrichtungen als Hydraulikdämpfer ausgebildet, mit denen es möglich ist, auf vergleichsweise kleinem Raum hohe Kräfte zu übertragen und darüber hinaus eine präzise Steuerung des Widerstandsverhaltens vorzunehmen. Ein Hydraulikdämpfer weist beispielsweise einen Verdrängerkolben auf, der in einem Zylinder verschieblich angeordnet ist und diesen in eine Extensionskammer und eine Flexionskammer teilt. Bei einer Relativbewegung von Oberteil zu Unterteil wird der Kolben innerhalb des Zylinders verlagert und verändert das Volumen der beiden Kammern, sodass Hydraulikfluid von der einen Kammer durch eine strömungstechnische Verbindung in die andere Kammer befördert wird. Zur Beeinflussung des Strömungsverhaltens und damit zur Beeinflussung des Widerstandsverhaltens sind in der strömungstechnischen Verbindung Drosseln oder Ventile eingebaut. Der Strömungswiderstand kann über die Drosseln oder Ventile eingestellt werden. Neben einer einmaligen Einstellung können die Strömungsquerschnitte auch während der Benutzung verändert werden, beispielsweise in Abhängigkeit von einem Gelenkwinkel oder auf der Grundlage von Sensorgrößen, die in einer elektronischen Steuerungseinrichtung verarbeitet werden.

Alternativ zu einer Linearhydraulik, bei der ein Kolben an einer Kolbenstange eine geradlinige Bewegung ausführt, existieren sogenannte Rotationshydrauliken, bei denen ein Kolben auf einem Zapfen angeordnet ist, der drehstarr mit einem der beiden Gelenkteile verbunden ist. An dem anderen Gelenkteil ist eine Kammer angeordnet oder ausgebildet, innerhalb der der Kolben verschwenkt wird. Der Kolben unterteilt die Kammer ebenfalls in eine Extensionskammer und eine Flexionskammer, die wiederum eine strömungstechnische Verbindung aufweisen. Eine solche Rotationshydraulik ist beispielsweise aus der DE 10 2017 124 337 B4 bekannt.

Die CN 216 478 131 U zeigt eine Rotationshydraulik mit einem Schwenkkolben, der an einem Zapfen angeordnet ist. Innerhalb des Schwenkkolbens und des Zapfens sind Nuten ausgebildet, in denen eine Dichtung angeordnet ist. Die Dichtung soll sicherstellen, dass kein Fluid durch den Spalt zwischen der Gehäusewandung und dem Schwenkkolben bzw. dem Drehzapfen hindurch tritt.

Die WO 99/00075 A1 betrifft eine computergesteuerte hydraulische Widerstandsvorrichtung für eine Prothese oder eine andere Vorrichtung mit einem Oberteil und einem schwenkbar daran gelagerten Unterteil und einem Rotor, der in einer Kammer schwenkbar angeordnet ist. Der Rotor weist zwei umlaufende Nuten mit umlaufenden Dichtungen auf.

Die US 2008/0255670 A1 betrifft ein Prothesen- oder Orthesengelenk mit einem ersten und einem zweiten Gelenkteil, die zueinander um eine Gelenkachse schwenkbar gelagert sind. Ein Schwenkkolben ist drehfest an einem der Gelenkteile angeordnet und in einer ein Fluid enthaltenden Verdrängerkammer geführt, wobei der Schwenkkolben die Verdrängerkammer in zwei Teilkammern unterteilt, die über einen Verbindungskanal zumindest über einen gewählten Verschwenkwinkelbereich des Schwenkkolbens miteinander verbunden sind. Zwischen dem Schwenkkolben und einer Verdrängerkammerwandung ist zumindest eine strömungsquerschnittbestimmende Kontur ausgebildet ist, die über einen gewählten Verschwenkwinkel in strömungstechnischer Verbindung mit dem Verbindungskanal und der Verdrängerkammer steht und in Abhängigkeit von der Winkelstellung des Schwenkkolbens einen unterschiedlichen freien Strömungsquerschnitt für den Durchtritt des Fluides bereitstellt.

Die DE 297 23 632 U1 betrifft eine Vorrichtung zum Erzeugen eines geregelten, veränderlichen Widerstands für ein Gelenk mit einer hydraulischen Betätigungseinrichtung, die ein Gehäuse aufweist, das ein bewegliches Element zum Aufbringen eines Widerstands einschließt, das innerhalb des Gehäuses eine erste Kammer von einer zweiten Kammer trennt. In einem Durchgang für das hydraulische Fluid von einer Kammer zu der anderen Kammer ist ein Solenoid-gesteuertes Ventil angeordnet, um die Strömung des hydraulischen Fluids zu regeln. Ein Sensor erfasst den Fluiddruck innerhalb jeder der Kammern. Ein Computersteuerungssystem betätigt das Ventil in Abhängigkeit der Sensorwerte, um die Strömung des Fluids durch das Ventil exakt zu regeln und um Veränderungen in der Vorrichtung und der Fluidviskosität zu kompensieren.

In anderen Ausgestaltungen wird die notwendige Abdichtung zwischen einer Extensionskammer und einer Flexionskammer durch möglichst geringe Spaltmaße bewirkt, um keine zusätzliche Dichtung um den Schwenkflügel herum zu benötigen. Die innere Dichtwirkung wird dabei über das Fertigen möglichst enger Spalte erzielt. Durch die Anwesenheit von Spalten kommt es zu sogenannten Spaltverlusten, die das übertragbare Moment der Gelenkeinrichtung begrenzen. Um die Spaltverluste zu minimieren, werden solche Rotationshydrauliken grundsätzlich mit einem Öl befüllt, das im Gegensatz zu dem Öl in Linearhydrauliken eine höhere Viskosität hat. Solche Hydraulikfluide verändern ihre Viskosität jedoch stark in Abhängigkeit von der Temperatur.

Aufgabe der vorliegenden Erfindung ist es, eine orthopädietechnische Gelenkeinrichtung bereitzustellen, mit der einerseits hohe Momente bis zu einer Sperrung der Gelenkeinrichtung übertragen werden können und andererseits das Niveau des Basiswiderstandes durch die Rotationshydraulik minimiert werden kann.

Gelöst wird diese Aufgabe durch eine orthopädietechnische Gelenkeinrichtung mit den Merkmalen des Hauptanspruches. Vorteilhafte Ausgestaltungen und Weiterbildungen der Erfindung sind in den Unteransprüchen, der Beschreibung sowie den Figuren offenbart.

Die orthopädietechnische Gelenkeinrichtung mit einem Oberteil und einem Unterteil, die schwenkbar um eine gemeinsame Gelenkachse aneinander gelagert sind, und einer ein Hydraulikfluid aufweisende Rotationshydraulik, die ein Gehäuse mit einer Kammer und einem darin schwenkbar gelagerten Schwenkkolben aufweist, der die Kammer in eine Flexionskammer und eine Extensionskammer unterteilt, die über zumindest einen Kanal hydraulisch miteinander verbunden sind, wobei der Schwenckolben eine Aufnahme aufweist, in der oder an der eine in Richtung auf das Gehäuse wirkende Dichtung angeordnet ist, zeichnet sich dadurch aus, dass die Dichtung ausgebildet ist, in einer ersten Schwenkrichtung durch das Hydraulikfluid mit einer von dem Gehäuse weg wirkenden Kraft beaufschlagt zu werden und in entgegengesetzter Verschwenkrichtung mit einer zu dem Gehäuse hin wirkenden Kraft beaufschlagt zu werden. Bevorzugt ist die Aufnahme für die Dichtung auf der Außenseite des Schwenkkolbens ausgebildet, wobei die Dichtung beispielsweise in einer als Nut ausgestalteten Aufnahme eingelegt ist. Alternativ ist die Aufnahme als ein den Schwenkkolben zumindest teilweise ausbildender Zapfen ausgebildet, an oder auf dem die Dichtung angeordnet wird. Orthopädietechnische Gelenkeinrichtungen haben je nach Einsatzzweck unterschiedliche Anforderungen. Beispielweise sollen häufig künstliche Kniegelenke in Gestalt von Prothesenkniegelenken oder Orthesenkniegelenken die Fähigkeit aufweisen, in Flexionsrichtung eine vollständige Sperrung zu ermöglichen, hingegen bei einer Streckung oder Extension einen möglichst geringen Widerstand der Verschwenkung entgegensetzen zu müssen. Künstliche Kniegelenke sollen die Möglichkeit bereitstellen, aus einer gebeugten, flektierten Erstellung möglichst schnell in eine gestreckte, extendierte Stellung gebracht zu werden, wohingegen eine möglichst vollständige Flexionssperrung möglich sein sollte, um ein unbeabsichtigtes Einbeugen oder ein Kollabieren des künstlichen Kniegelenkes zu vermeiden. Bei künstlichen Ellenbogengelenken kann eine Extensionssperre bei gleichzeitiger leichter Einbeugbarkeit gewünscht sein. Bei künstlichen Knöchelgelenken können ein hoher Widerstand gegen eine Dorsalflexion und ein geringer Widerstand gegen eine Plantarflexion vorteilhaft sein. Da für eine vollständige Sperrung eine zwischen dem Schwenkkolben und der Gehäusewand wirksame Dichtung als Bauteil vorteilhaft ist, um Spaltverluste zu vermeiden, liegt die Dichtung an der Gehäusewand an und ist in einer Ausgestaltung in Richtung auf die Gehäusewand vorgespannt. Dabei ist die Dichtung bzw. der Dichtungsquerschnitt so ausgebildet, dass in einer ersten Verschwenkrichtung ein gegen die Dichtung drückendes Hydraulikfluid, das in den Spalt zwischen dem Schwenkkolben und dem Gehäuse eindringt, die Dichtung von der Gehäusewand weg drückt, um so einen beabsichtigten Spaltverlust zu erzeugen. Dadurch wird der systembedingte Widerstand gegen eine Verschwenkung in diese Richtung, beispielsweise Extensionsrichtung, minimiert, da zusätzlich zu der Strömung durch den Verbindungskanal zwischen der Extensionskammer und der Flexionskammer Hydraulikfluid durch den Spalt hindurch treten kann bzw. die Reibung der Dichtung an der Gehäusewand verringert oder aufgehoben wird. In der entgegengesetzten Richtung, beispielsweise Flexionsrichtung, wird hingegen durch den Druck des Hydraulikfluides innerhalb des Spaltes die Dichtlippe gegen die Gehäusewandung gedrückt, wodurch der Anpressdruck der vorzugsweise elastisch gegen die Gehäusewand vorgespannten Dichtung erhöht wird. Es tritt somit eine selbstverstärkende, richtungsabhängige Dichtwirkung auf, mit der eine vollständige Abdichtung ohne Spaltverluste bei einem vollständig geschlossenen hydraulischen Verbindungskanal zwischen der Extensionskammer und der Flexionskammer erreicht werden kann. Die Dichtung zwischen der Gehäusewand und dem Schwenckolben ist dabei vorzugsweise über den gesamten äußeren Umfang des Schwenckolbens herum ausgebildet und erstreckt sich sowohl an den von einem Drehzapfen radial nach außen verlaufenden, axial zueinander beabstandeten Seitenkanten als auch an der radial äußeren Kopfseite.

In einer Ausgestaltung ist die Dichtung in Richtung auf das Gehäuse vorgespannt, insbesondere elastisch vorgespannt, beispielsweise durch die Materialbeschaffenheit der Dichtung, die insbesondere flexibel und/oder elastisch verformbar ist oder beispielsweise durch ein separates, federndes Element, zum Beispiel eine Feder, ein Elastomerelement oder dergleichen, wodurch die Dichtung gegen die Gehäusewand vorgespannt wird. In einer Ausgestaltung weist die Dichtung ein Übermaß auf und wird bei der Montage komprimiert, um sich während der Benutzung an die Gehäusewand anzulegen.

In einer Ausgestaltung weist die Dichtung zumindest eine Dichtlippe auf, an der zumindest ein Kontaktbereich zur Anlage an dem Gehäuse ausgebildet ist, wobei die Dichtlippe eine geneigte Kontur aufweist. Die Neigung der Dichtlippe verläuft schräg von der Flexionskammer zur Extensionskammer, also schräg zu der Verschwenkrichtung. In dem Bereich der Kopfseite bzw. Verbindungskante oder radial äußeren Kante des Schwenkkolbens weist die Dichtlippe eine zur Radialrichtung geneigte Kontur auf. Die Richtung der Neigung ist dabei abhängig von dem gewünschten Verhalten der Dichtung während der Benutzung. Um eine Dichtungsverstärkung der Dichtlippe bei einer Flexionsbewegung zu erreichen, ist die Dichtlippe von der Extensionskammer in Richtung auf die Flexionskammer mit einer in Richtung auf die Gehäusewand gerichteten Steigung versehen, sodass das der Flexionskammer zugewandte Ende der Dichtung in einem unbelasteten Zustand weiter weg von dem Schwenkkolben befindlich ist als das der Extensionskammer zugewandte Ende. Soll die verstärkende Wirkung des Hydraulikfluides genutzt werden, um bei einer Extensionsbewegung die Dichtung gegen die Gehäusewand zusätzlich zu einer bereits vorhanden Anpresskraft zu beaufschlagen, ist die Richtung der Neigung umgekehrt. Die Neigung oder Steigung kann kontinuierlich, linear oder aber auch in Stufen ausgebildet sein.

In einer Weiterbildung weist die Dichtlippe einen Radialabschnitt auf, der sich an die Dichtlippe anschließt und sich von der Dichtlippe nach innen in Richtung auf den Schwenkkolben oder die Aufnahme erstreckt und sich an einer Aufnahmewand oder an einem Träger abstützt. Die Aufnahmewand ist in einer Ausgestaltung der Aufnahme als eine Nut eine der Nutwandungen. Wird die Dichtung an der Außenseite der Aufnahme angeordnet, ist dafür ein Träger vorgesehen, an dem sich die Dichtlippe abstützt. Der Träger kann beispielsweise als ein Überzug oder eine Kappe ausgebildet sein, der auf einen Kern oder einen Zapfen des Schwenkkolbens aufgesteckt wird.

In einer Weiterbildung weist die Dichtung einen Basisabschnitt auf, der sich an den Radialabschnitt anschließt und sich auf dem Aufnahmegrund oder dem Träger abstützt bzw. als Träger ausgebildet ist. Bei Ausgestaltung der Aufnahme als Nut befindet sich der Radialabschnitt in dem Nutgrund oder stützt sich darauf ab, ist der Träger ein Aufsatz oder Überzug, wird der Basisabschnitt an dem Träger befestigt oder ausgebildet.

Zwischen dem Basisabschnitt und der Dichtlippe ist in einer Ausgestaltung ein Stauraum für das Hydraulikfluid ausgebildet, in den das Hydraulikfluid eindringen kann, wenn der Schwenkkolben in eine Richtung bewegt wird, in der das Hydraulikfluid die Dichtlippe in Richtung auf das Gehäuse presst oder eine unterstützende Kraft ausübt. Durch das Sammeln des Hydraulikfluides in dem Stauraum und die Gestaltung des Stauraumes ist es möglich, die Krafteinleitung und Verformung der Dichtung bei zunehmendem Staudruck zu beeinflussen. Beispielsweise ist es möglich, mehrere in Verschwenkrichtung hintereinander angeordnete Kontaktbereiche nacheinander oder kaskadiert und in Abhängigkeit von dem auf die Dichtung wirkenden Druck nach außen in Richtung auf das Gehäuse zu verlagern, um eine mehrfache Abdichtung durch die Dichtung zu ermöglichen.

Insbesondere zur Vermeidung einer unbeabsichtigten Einbeugung ist die Dichtlippe in Richtung auf die Flexionskammer nach außen geneigt oder nach außen schräg orientiert ausgebildet, um eine Flexionssperre zu ermöglichen. Ist eine Extensionssperre gewünscht, ist eine entsprechend umgekehrte Neigung bzw. Schrägstellung verwirklicht.

Die Dichtlippe ist in einer Ausführungsform in Abhängigkeit von der Schwenkrichtung einseitig in Radialrichtung druckverstärkend und druckvermindernd orientiert ausgebildet, wobei die Druckverstärkung in der einen Schwenkrichtung und die Druckminderung in der entgegengesetzten Verschwenkrichtung auftritt und sich auf den Anpressdruck der Dichtlippe durch das Hydraulikfluid bezieht. Die Verstärkung oder Verminderung erfolgt in Richtung auf das Gehäuse, sodass die Dichtlippe oder Dichtung von dem Schwenkkolben weg in Richtung auf die Gehäusewand oder umgekehrt von der Gehäusewand weg in Richtung auf den Schwenkkolben mit einer Kraft beaufschlagt wird.

**In** einer Ausgestaltung sind mehrere Dichtlippen oder Kontaktbereiche hintereinander angeordnet, die kaskadiert druckaktivierbar ausgebildet sind. Eine Verstärkung der Dichtwirkung tritt zunächst bei der in Verschwenkrichtung vorderen Dichtlippe oder dem vorderen Kontaktbereich auf, bei zunehmendem Druck wird dann ein weiterer Kontaktbereich oder eine weitere Dichtlippe an die Gehäusewand gepresst oder der Anpressdruck verstärkt. Umgekehrt wird bei einer den Anpressdruck der Dichtlippe vermindernden Verschwenkrichtung zunächst die vorderste Dichtlippe oder der vorderste Kontaktbereich von der Gehäusewand wegbewegt oder mit einer davon wegweisenden Kraft beaufschlagt, bevor dann der in Verschwenkrichtung dahinterliegenden Kontaktbereich oder die dahinter liegende Dichtlippe entsprechend verlagert oder verformt wird.

Die Aufnahme der Dichtung ist in einer Ausgestaltung an den Seitenkanten und der Kopfseite des Schwenkkolbens ausgebildet, beispielsweise als Nut. Alternativ ist die Aufnahme als Zapfen ausgebildet, um den Träger der Dichtung aufzunehmen.

Die Aufnahme kann auch in einem Drehzapfen ausgebildet sein, an dem der Schwenkkolben angeordnet oder angeformt ist.

In einer Ausgestaltung ist der Schwenkkolben mehrteilig ausgebildet, um die Fertigung und Montage zu erleichtern. Zwischen zwei Schwenkkolbenteilen ist dann die Aufnahme ausgebildet.

An dem Schwenkkolben ist in einer Ausgestaltung in oder an der Aufnahme zumindest ein Formschlusselement ausgebildet oder angeordnet, das in dem montierten Zustand mit einem an der Dichtung korrespondierend ausgebildeten Formschlusselement wechselwirkt. Das Formschlusselement an oder in der Aufnahme kann beispielsweise als eine Ausnehmung in eine Aufnahmewand oder einem Aufnahmegrund bzw. an dem Zapfen ausgebildet sein, wohingegen an der Dichtung einen entsprechend geformter Vorsprung angeformt oder ausgebildet oder angeordnet ist. Umgekehrt können die Formschlusselemente an dem Schwenkkolben als Vorsprung und an der Dichtung als entsprechend geformte Ausnehmung ausgebildet sein. Die Formschlusselemente sind so angeordnet, dass sie in dem monierten Zustand der Dichtung in oder an der Aufnahme miteinander in Eingriff treten.

Die orthopädietechnische Gelenkeinrichtung ist in einer Ausgestaltung als künstliches Kniegelenk oder Knöchelgelenk, insbesondere als Prothesenkniegelenk bzw. Prothesenknöchelgelenk oder Orthesenkniegelenk oder Orthesenknöchelgelenk oder als Exoprothese oder Orthese für die Hüfte, die Hand oder andere Körperteile ausgebildet.

In dem Kanal, der die hauptsächliche strömungstechnische Verbindung zwischen der Extensionskammer und der Flexionskammer ausbildet, ist vorteilhafterweise zumindest eine Drossel oder ein Ventil angeordnet, über das der Strömungswiderstand zwischen der Extensionskammer und der Flexionskammer eingestellt werden kann. Das Ventil oder die Drossel können permanent eingestellt sein, wobei die Verstellung zur Anpassung an die gewünschten Widerstandseigenschaften der Rotationshydraulik angepasst werden kann. Alternativ ist eine insbesondere mikroprozessorgesteuerte, sensorbasierte Steuerung in Abhängigkeit von Sensorwerten möglich, bei der während der Benutzung und/oder Bewegung der orthopädietechnischen Gelenkeinrichtung Daten gesammelt und auf Grundlage dieser Daten über einen Aktuator die Drossel oder das Ventil verstellt wird.

Die Dichtung weist in einer Ausgestaltung eine die Reibung reduzierende Beschichtung oder Oberflächenbehandlung auf. Dadurch wird die Verschwenkbewegung in einer Richtung, in der die Dichtung von der Gehäusewand wegbewegt oder mit einer entsprechend gerichteten Kraft beaufschlagt wird, weiter erleichtert und der Widerstand dagegen weiter reduziert. Durch die reibungsreduzierende Beschichtung oder Oberflächenbehandlung ist somit eine leichte Verschwenkbarkeit der orthopädietechnischen Gelenkeinrichtung erreichbar. Der reibungsreduzierende Effekt durch die Beschichtung oder Oberflächenbehandlung wird in der entgegengesetzten Schwenkrichtung durch die Geometrie und die durch den Hydraulikdruck verstärkte Dichtwirkung ausgeglichen und überkompensiert. Als Oberflächenbehandlung ist beispielsweise insbesondere eine topografieändernde Behandlung wie die RFN-Behandlung (Reduced Friction by Nanotechnology) vorgesehen.

Nachfolgend werden Ausführungsbeispiele der Erfindung anhand der Figuren näher erläutert. Gleiche Bezugszeichen bezeichnen gleiche Komponenten. Es zeigen:
- Figur 1: eine Darstellung eines Teils eines Orthesenkniegelenkes;
- Figur 2 -: eine Detaildarstellung eines Rotationskolbens;
- Figur 3 -: eine Einzeldarstellung einer Dichtung;
- Figur 4 -: eine Variante der Figur 2;
- Figur 5 -: eine Einzeldarstellung der Dichtung in der Figur 4;
- Figur 6 -: eine perspektivische Darstellung einer Variante der Figur 1;
- Figur 7 -: eine Einzeldarstellung eines einteiligen Schwenkkolbens;
- Figur 8 -: eine Einzeldarstellung einer Dichtung gemäß Figur 7;
- Figur 9 -: eine Schnittdarstellung durch eine Dichtung;
- Figur 10 -: eine perspektivische Darstellung einer Dichtung gemäß Figur 9;
- Figur 11 -: eine Variante eines Schwenkkolbens; sowie
- Figur 12 -: eine Seitenansicht einer orthopädietechnischen Gelenkeinrichtung.

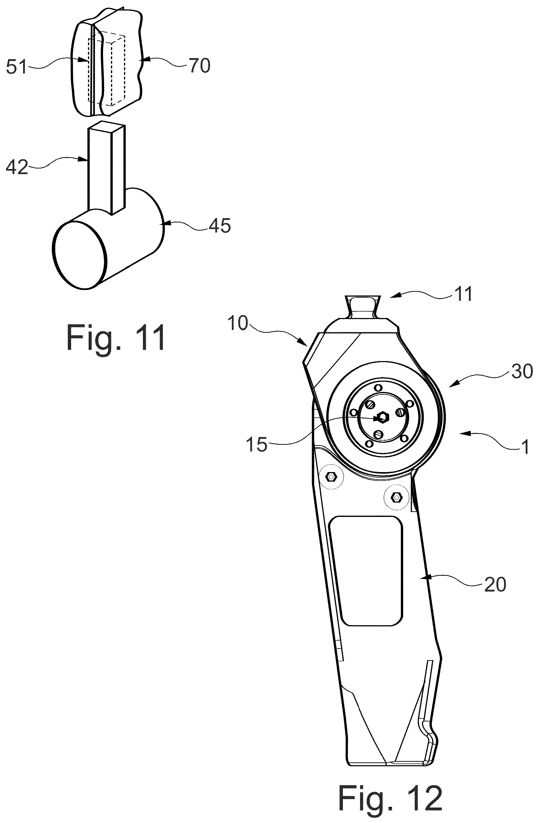

**In** Figur 12 ist in einer Seitenansicht eine orthopädietechnische Gelenkeinrichtung in Gestalt eines Exoprothesenkniegelenkes 1 dargestellt, das ein Oberteil 10 mit oberen Anschlussmitteln 11 in Gestalt eines Pyramidenadapters aufweist. An dem oberen Anschlussmittel 11 kann ein Prothesenschaft zur Aufnahme eines Oberschenkelstumpfes befestigt werden. Das Oberteil 10 ist um eine Schwenkachse 15 schwenkbar um ein Unterteil 20 gelagert, an dessen distalem Ende eine Aufnahme für ein Unterschenkelrohr ausgebildet ist. Innerhalb des Unterteils 20 ist ein Gehäuse 30 ausgebildet oder angeordnet, in dem eine Rotationshydraulik untergebracht ist. Innerhalb des Unterteils 20 können weitere Komponenten der Rotationshydraulik angeordnet sein.

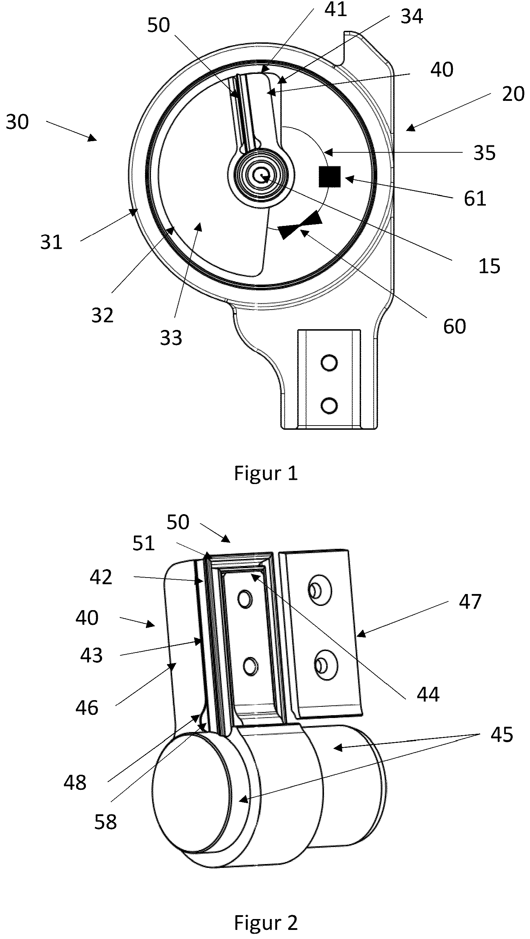

Figur 1 zeigt in einer Seitenansicht einen Teil eines Orthesenkniegelenkes 1 mit einem Gehäuse 30, in dem eine Kammer 32 ausgebildet ist, in der ein Schwenkkolben 40 schwenkbar um die Schwenkachse 15 gelagert ist. Das Orthesenkniegelenk 1 ist als monozentrisches Kniegelenk ausgebildet, und der Schwenkkolben 40 ist drehfest mit dem Oberteil 10 verbunden. In der dargestellten Position gemäß Figur 1 befindet sich der Schwenkkolben 40 in der Extensionsendlage, in der das Orthesenkniegelenk eine maximale Streckung erreicht hat. Der Schwenkkolben 40 unterteilt die Kammer 31 in eine Extensionskammer 34 und eine Flexionskammer 33. Wird das Oberteil 10 relativ zu dem Unterteil 20 in Flexionsrichtung verschwenkt, wird aufgrund der drehstarren Kopplung des Schwenkkolbens 40 mit dem Oberteil 10 der Schwenkkolben 40 entgegen dem Uhrzeigersinn innerhalb der Kammer 32 verschwenkt. Hydraulikfluid, das in der Kammer 32 befindlich ist, wird von der Flexionskammer 33 durch den Kanal 35 in dem Gehäuse 30 und durch eine Drosseleinrichtung in Gestalt eines Ventils 61 und/oder einer Drossel 60 in die Extensionskammer 34 bewegt. Das Ventil 61 kann verstellbar oder einstellbar ausgebildet sein. Eine Verstellbarkeit kann durch eine Computersteuerung auf Basis von Sensoren erfolgen. Alternativ kann die Drossel 60 und/oder das Ventil 61 dauerhaft auf den jeweiligen Nutzer eingestellt werden. Zur Verstellung können über einen Zugang von außen die Drossel 60 oder das Ventil 61 manuell und dauerhaft verstellt werden. Eine solche Gelenkeinrichtung oder ein solches Gelenk 1 ist in einer Ausgestaltung Teil einer Orthese, zwei Gelenkeinrichtungen, die medial und lateral an dem Knie angeordnet sind, können in einer Orthese kombiniert werden. Alternativ ist nur eine solche Gelenkeinrichtung monolateral an dem Knie angeordnet. Distal und proximal besitzt das Orthesengelenk in diesem Fall vorzugsweise Schienenaufnahmen, um das Gelenk mit den restlichen Bauteilen der Orthese zu verbinden. Auch die Anwendung an anderen Gelenkstellen, beispielsweise an einem Ellenbogengelenk, ist in einer Ausgestaltung verwirklicht. Alternativ wird die Schwenkkolbenanordnung in einem Prothesenkniegelenk eingesetzt.

In der Figur 1 ist der Schwenkkolben 40 einteilig ausgebildet und weist eine nutartige Aufnahme für die Dichtung 50 auf. Die Dichtung 50 ist in der Nut eingesetzt. Die Dichtung 50 verläuft umlaufend an der gesamten Außenseite 41 des Schwenkkolbens 40 und hat eine Dichtlippe 51, die in Richtung auf das Gehäuse 31 über die Außenseite 41 hinaus steht. Die Aufnahme wird durch die Dichtung 50 ausgefüllt und stützt die Dichtung 50 in beiden Schwenkrichtungen an jeweils einer Aufnahmewand ab. Das Widerlager zum Abstützen der Dichtung 50, damit diese in Richtung auf das Gehäuse 31 wirken kann, wird durch den Aufnahmegrund oder den Nutgrund ausgebildet.

In der Figur 2 ist der Schwenkkolben 40 gemäß der Figur 1 in einer Einzeldarstellung gezeigt. Der Schwenkkolben 40 weist zwei Schwenkkolbenteile 46, 47 auf, die lösbar miteinander verbunden sind. Die Befestigung kann beispielsweise über Schrauben erfolgen, die durch Bohrungen in einem Schwenkkolbenteil 47 hindurchgeführt und in Gewinde innerhalb des anderen Schwenkkolbenteils 46 eingeschraubt werden können. Innerhalb der Schwenkkolbens 41 ist eine Ausnehmung als Formschlusselement 48 eingearbeitet, in das ein korrespondierendes Formschlusselement 58 als nasenförmiger Vorsprung formschlüssig eingreifen kann. Die Dichtung 50 ist in der zwischen den beiden Schwenkkolbenteilen 46, 47 gebildeten Aufnahme 42 eingelegt und wird darin über die Formschlusselemente 48, 58 in radialer Richtung gesichert. Die durch die Schwenkkolbenteile 46, 47 ausgebildeten Aufnahmewände 43 sichern die Dichtung 50 gegen eine Verlagerung bei einer Verschwenkung in beiden Verschwenkrichtungen, an die Aufnahmewände 43 schließt sich ein Aufnahmegrund 44 an, auf dem sich die Dichtung 50 innen abstützt. Die Dichtung 50 weist eine über die Außenkontur oder Außenseite 41 des Schwenkkolbens 40 hinausstehende Dichtlippe 51 auf, deren Aufbau und Funktion später erläutert wird. Der Schwenkkolben 40 ist drehfest zwischen zwei Drehzapfen 45 gelagert, die sich in einer Verlagerung in dem Unterteil 20 drehen und die starr mit dem nicht dargestellten Oberteil 10 gekoppelt sind. Dadurch wird bei einer Verschwenkbewegung des Oberteils 10 gegenüber dem Unterteil 20 eine Verschwenkung des Schwenkkolbens 40 um die Gelenkachse 15 bewirkt. Die Bezeichnung "Oberteil" und "Unterteil" beinhaltet keine Einschränkung hinsichtlich der Anordnung der Komponenten, insbesondere keine Bestimmung der Anordnung in distaler oder proximaler Zuordnung zueinander, sondern dient nur zur Unterscheidung der Komponenten.

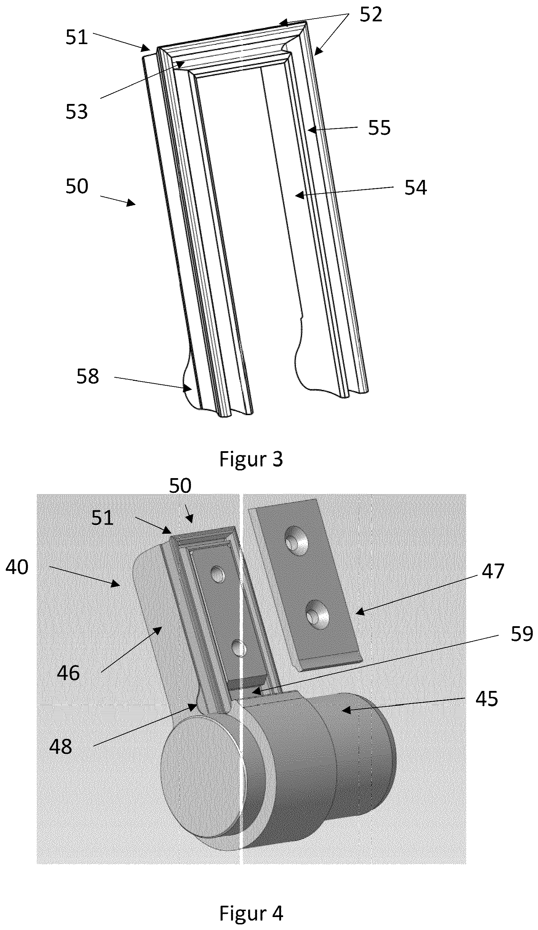

**In** der Figur 3 ist die Dichtung 50, die in dem Schwenkkolben gemäß der Figur 2 eingelegt ist, in Einzeldarstellung dargestellt. Die Dichtung 50 weist eine im Wesentlichen U-förmige Kontur auf, mit dem Verbindungschenkel an der Kopfseite oder radial äußeren Verbindungskante zwischen den beiden radial nach außen sich erstreckenden Seitenschenkeln, die an den Seitenkanten des Schwenkkolbens angeordnet werden. An den Enden der freien Seitenschenkel sind jeweils Formschlusselemente 58 ausgebildet, die als Vorsprünge ausgebildet sind und in die korrespondierenden Ausnehmungen 48 in dem Schwenkkolbenteil 46 eingesetzt werden. Die Dichtung 50 weist eine außen umlaufende Dichtlippe 51 auf, die zumindest einen Anlagebereich 52 aufweist, der sich in dem montierten Zustand an die Gehäusewand angelegt. Die Dichtlippe 51 mit den Kontaktbereichen 52 erstreckt sich in Verschwenkrichtung über eine Tiefe von der Aufnahmewand bis zu dem freien Ende der Dichtlippe 51, wobei sich an die Dichtlippe 51 ein Radialabschnitt 53 anschließt, der sich von der Dichtlippe 51 oder der Außenkontur 41 des Schwenkkolbens 40 nach innen erstreckt. Der Radialabschnitt 53 verläuft an der Kopfseite radial nach innen, an den Seitenkanten des Schwenkkolbens in axialer Richtung nach innen in Richtung auf den Schwenkkolben bzw. an einer Aufnahmewand entlang. An den Radialabschnitt 53 schließt sich ein Basisabschnitt 54 an, der sich auf dem Aufnahmegrund 44 abstützt. Die Tiefe des Basisabschnittes 54 kann größer als die Tiefe der Dichtlippe 51 sein. Zwischen dem Basisabschnitt 54, dem Radialabschnitt 53 und der Dichtlippe 51 ist ein Stauraum 55 ausgebildet, in den Hydraulikfluid von einer Kammer aus eindringen kann, wenn der Schwenkkolben 40 in die entsprechende Richtung verdreht wird. Hydraulikfluid kann von der Seite des angeschraubten Schwenkkolbenteils 47 von der Dichtlippe 51 aufgrund einer geneigten Ausgestaltung nach innen in Richtung auf den Schwenkkolben 40 in den Stauraum 55 geleitet werden. Aufgrund des sich in dem Stauraum 55 gestauten Hydraulikfluides wird das Material der Dichtung 50 komprimiert und nach außen gedrückt. Der Basisabschnitt 54 wird gegen den Aufnahmegrund 44 gedrückt, die Dichtlippe 51 mit den Kontaktbereichen 52 gegen die Gehäusewandung. Dadurch wird der Anpressdruck der Dichtlippe 51 und der Kontaktbereiche 52 gegenüber der Gehäusewandung vergrößert und eine verbesserte Dichtwirkung erzielt.

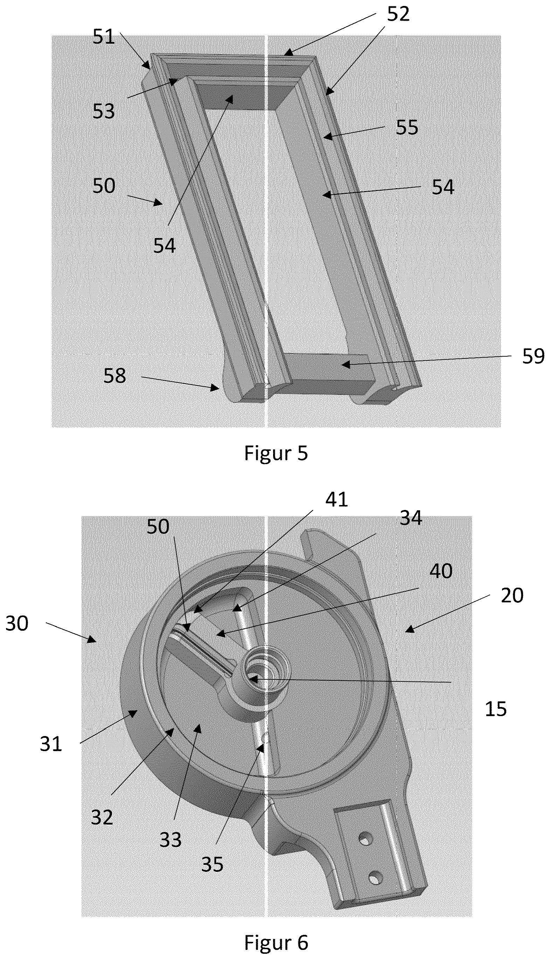

In der Figur 4 ist eine Variante der mehrteiligen Ausgestaltung des Schwenkkolbens gemäß der Figur 2 dargestellt. Der Unterschied zu der Ausführungsform gemäß Figur 2 besteht darin, dass an dem Übergang des Schwenkkolbens 40 zu dem Drehzapfen 45 eine Nut in den Schwenkkolben 40 eingearbeitet ist, in dem ein Steg 59 der Dichtung 50 eingelegt wird. Die Ausgestaltung der entsprechenden Dichtung 50 ist in der Figur 5 gezeigt. Der Steg 59 verbindet die beiden Seitenschenkel der Dichtung 50, die an den Seitenkanten des Schwenkkolbens die Dichtwirkung zwischen der Extensionskammer und der Flexionskammer bewirken. Der Quersteg 59 stabilisiert die nunmehr im Wesentlichen rechteckige Dichtung 50 und verhindert zusätzlich eine ungewollte Verformung der Dichtung 50.

Eine Variante der Ausgestaltung der Figur 1 ist in der Figur 6 gezeigt, bei der der Schwenkkolben 40 eine andere Kontur aufweist und darüber hinaus einteilig ausgebildet ist. In der Figur 6 ist auch der Kanal 35 in dem Unterteil 20 gezeigt, der eine strömungstechnische Verbindung zwischen der Extensionskammer 34 und der Flexionskammer 33 herstellt. Innerhalb dieses Kanales 35 sind die Einrichtungen zur Beeinflussung des Strömungswiderstandes angeordnet, insbesondere zumindest eine Drossel oder ein Ventil.

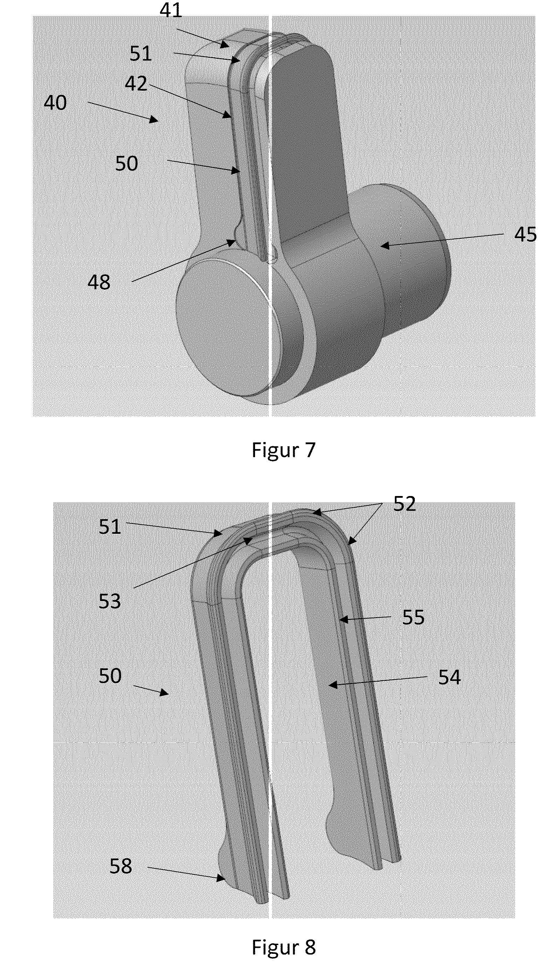

Die Figur 7 zeigt den Schwenkkolben 40 in der Verwendung gemäß der Figur 6 in einer Einzeldarstellung. Der Schwenkkolben 40 weist eine abgerundete Außenkontur auf, die Aufnahme 42 ist als eine eingefräste oder eingearbeitet Nut ausgebildet, in der die im Wesentlichen U-förmige Dichtung 50 eingesetzt ist. Die Dichtlippe 51 steht über die Außenseite 41 des rückwärtigen Teils des Schwenkkolbens 40 hinaus, die Vorderseite des Schwenkkolbens 40 ist mit einer leicht verkleinerten Kontur oder einer Abschrägung versehen, sodass auf der dem Radialabschnitt gegenüberliegenden Seite ein größeres Spaltmaß zwischen dem Kolben und der Gehäusewand vorhanden ist als auf der Seite der Aufnahmewand, an der sich der Radialabschnitt abstützt. Dadurch wird erreicht, dass Hydraulikfluid leichter in den Stauraum 55 eindringen und die Dichtlippe 51 nach außen an die Gehäusewand pressen kann.

In der Figur 8 ist die gebogene Form der Dichtung 50, die in dem Schwenkkolben 40 der Figur 7 eingesetzt ist, gezeigt, der grundsätzliche Aufbau entspricht dem der Dichtungen 50 in der Figuren 3 und 5.

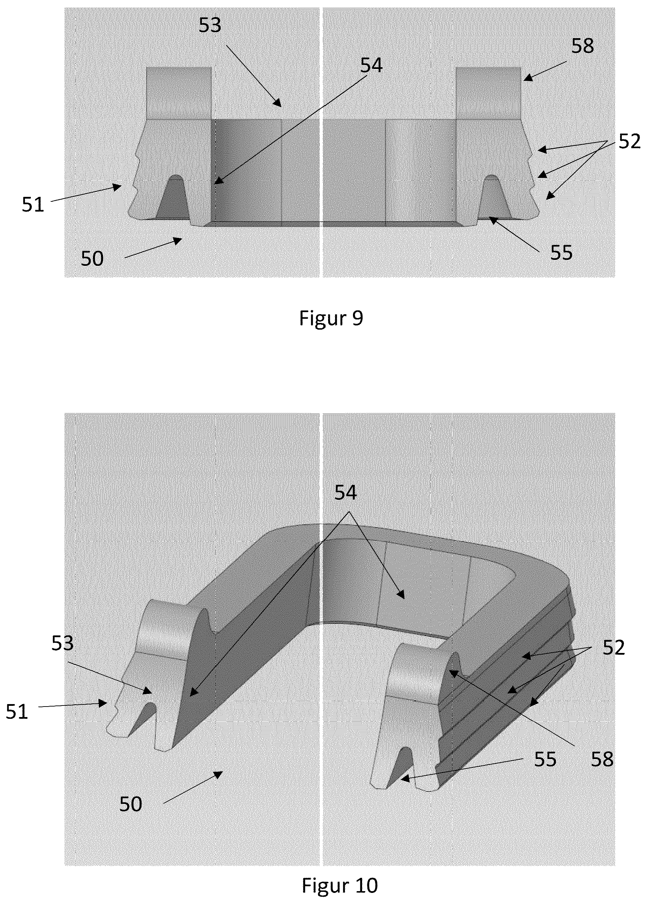

In der Figur 9 ist eine Querschnittsdarstellung der Dichtung 50 gezeigt. Die Rückseite mit den Formschlusselementen 58 und dem Radialabschnitt 53 ist oben, der Stauraum 55 öffnet sich nach unten. Die Dichtung 55 weist eine umlaufende, außenliegende Dichtlippe 51 mit insgesamt drei Anlagebereichen 52 auf, die stufenförmig ausgebildet sind und Dichtkanten aufweisen, die sich an die nicht dargestellte Gehäusewand anlegen können, wobei in der Ausgangsstellung ohne Druckbeaufschlagung nicht alle Dichtkanten anliegen müssen. Das Anlegen einer Dichtkante an der Gehäusewand ist vorteilhaft, um den Aufbau des Anpressdruckes in dem Stauraum zu erleichtern, ist aber nicht unbedingt notwendig. Die Dichtlippe kann sich auch erst nach dem Aufbau eines Staudruckes an die Gehäusewand anlegen. Der Basisabschnitt 54 steht über die Vorderkante der Dichtlippe 51 hinaus, sodass sich zudem ein Spalt in Verschwenkrichtung einstellt, durch den Hydraulikfluid in den Stauraum 55 eintreten kann.

In der Figur 10 ist in einer perspektivischen Darstellung die Dichtung 50 gemäß Figur 9 dargestellt. In beiden Darstellungen ist zu erkennen, dass der Basisabschnitt 54 eine größere Tiefe als die Dichtlippe 51 hat und über die Vorderkante der Dichtlippe 51 hinaussteht, sodass sich ein Spalt in Verschwenkrichtung einstellt, durch den Hydraulikfluid in den Stauraum 55 eintreten kann. Wird die Dichtung 50 gemäß der Darstellung der Figuren 9 und 10 in der Bildebene nach unten verschwenkt, biegt sich die Dichtlippe 51, deren vorderer Kontaktbereich 52 bereits an der Gehäusewand anliegen kann, weiter in Richtung an die Gehäusewand, da der Staudruck und insbesondere der Kontaktbereich 52 einen Widerstand gegen die Verschwenkbewegung aufbaut und das flexible, insbesondere elastische Material der Dichtlippe 50 nach außen aufrollt, um so die Breite des Stauraumes 55 an der Vorderkante der Dichtlippe 51 zu vergrößern. Gleichzeitig sorgt das mit hohem Druck in den Stauraum 55 eindringende Hydraulikfluid dafür, dass das Material der Dichtlippe 50 gegen den Aufnahmegrund und gegen die Gehäusewand gedrückt wird. Dadurch legt sich der der Vorderkante oder dem ersten Kontaktbereich 52 nachfolgende Kontaktbereich 52 an die Gehäusewand an, danach folgt der dritte Kontaktbereich 52, der am nächsten den Formschlusselementen 58 liegt. Die Kontaktbereiche 52 sind jeweils Kanten, die an sägezahnartig ausgebildeten Oberflächen der Außenseite der Dichtlippe 51 ausgebildet sind. Die Neigung der stufenförmig ausgebildeten Wandabschnitte erstreckt sich von dem Radialabschnitt 53 geneigt nach außen, sodass Hydraulikfluid, das von der Seite des Radialabschnittes 53 gegen die Dichtlippe 51 drückt, das Material der Dichtung 50 in Richtung auf den Basisabschnitt 54 bzw. Aufnahmegrund 44 drückt. Dadurch wird Kontaktbereich 52 von der Gehäusewand wegbewegt und die Dichtlippe 51 insbesondere in dem Bereich des Stauraumes 52 nach innen in Richtung auf den Schwenkkolben 40 gebogen, wodurch Hydraulikfluid durch einen Spalt an der Außenseite des Schwenkkolbens entlang der Dichtung 50 vorbei in die andere Kammer eintreten kann. Dadurch verringert sich der Widerstand gegen die entsprechende Rotationsbewegung, insbesondere gegen eine Extensionsbewegung.

In der Figur 11 ist eine Variante gezeigt, bei der der Schwenkkolben eine zapfenförmige Aufnahme 42 aufweist, auf die ein Träger 70 aus einem flexiblen, insbesondere elastische Material aufgesteckt wird. An der Außenseite des Trägers 70 ist dann die Dichtlippe 51 angeordnet oder angeformt, deren Kontur in einer Verschwenkrichtung von dem Träger 70 weg in Richtung auf die Gehäusewand ansteigend geneigt ist, sodass eine schwenkrichtungsabhängige Erhöhung der Anpresskraft der Dichtung 50 an die Gehäusewand bewirkt wird. In der einen Richtung wird die Dichtlippe 51 mit einer von dem Gehäuse weg wirkenden Kraft beaufschlagt, insbesondere von der Gehäusewand abgehoben, in der anderen Richtung wird die Dichtlippe 51 durch das Hydraulikfluid und gegebenenfalls durch eine Verformung aufgrund der Reibbewegung an dem Gehäuse in Richtung auf das Gehäuse mit einer verstärkenden Kraft beaufschlagt.

Die Dichtung 50 und insbesondere die Dichtlippe 51 kann mit einer reibungsverminderten Beschichtung oder Oberflächenbehandlung versehen sein, um insbesondere in der Verschwenkrichtung, bei der eine verminderte Anpresswirkung bereitgestellt wird, einen weiter verminderten Reibungswiderstand bereitzustellen.

## Patentansprüche

1. Orthopädietechnische Gelenkeinrichtung mit einem Oberteil (10) und einem Unterteil (20), die schwenkbar um eine gemeinsame Gelenkachse (15) aneinander gelagert sind, und einer ein Hydraulikfluid aufweisende Rotationshydraulik (30), die ein Gehäuse (31) mit einer Kammer (32) und einem darin schwenkbar gelagerten Schwenkkolben (40) aufweist, der die Kammer (32) in eine Flexionskammer (33) und eine Extensionskammer (34) unterteilt, die über zumindest einen Kanal (35) hydraulisch miteinander verbunden sind, wobei der Schwenckolben (40) eine Aufnahme (42) aufweist, in oder an der eine in Richtung auf das Gehäuse (31) wirkende Dichtung (50) angeordnet ist, **dadurch gekennzeichnet, dass** die Dichtung (50) ausgebildet ist, in einer ersten Schwenkrichtung durch das Hydraulikfluid mit einer von dem Gehäuse (31) weg wirkenden Kraft beaufschlagt zu werden und in entgegengesetzter Schwenkrichtung mit einer zu dem Gehäuse (31) hin wirkenden Kraft beaufschlagt zu werden.

2. Orthopädietechnische Gelenkeinrichtung nach Anspruch 1, **dadurch gekennzeichnet, dass** die Dichtung (50) in Richtung auf das Gehäuse (31) vorgespannt, insbesondere elastisch vorgespannt ist.

3. Orthopädietechnische Gelenkeinrichtung nach Anspruch 1 oder 2, **dadurch gekennzeichnet, dass** die Dichtung (50) zumindest eine Dichtlippe (51) aufweist, an der zumindest ein Kontaktbereich (52) zur Anlage an dem Gehäuse (31) ausgebildet ist und die Dichtlippe (51) eine geneigte Kontur aufweist.

4. Orthopädietechnische Gelenkeinrichtung nach Anspruch 3, **dadurch gekennzeichnet, dass** sich an die Dichtlippe (51) ein Radialabschnitt (53) anschließt, der sich von der Dichtlippe (51) nach innen erstreckt und sich an einer Aufnahmewand (43) oder einem Träger (70) abstützt.

5. Orthopädietechnische Gelenkeinrichtung nach Anspruch 4, **dadurch gekennzeichnet, dass** die Dichtung (50) einen Basisabschnitt (54) aufweist, der sich an den Radialabschnitt (53) anschließt und sich auf dem Aufnahmegrund (44) oder dem Träger (70) abstützt.

6. Orthopädietechnische Gelenkeinrichtung nach Anspruch 5, **dadurch gekennzeichnet, dass** zwischen dem Basisabschnitt (54) und der Dichtlippe (51) ein Stauraum (55) für Hydraulikfluid ausgebildet ist.

7. Orthopädietechnische Gelenkeinrichtung nach einem der Ansprüche 3 bis 6, **dadurch gekennzeichnet, dass** die Dichtlippe (51) in Richtung auf die Flexionskammer (33) geneigt ausgebildet ist.

8. Orthopädietechnische Gelenkeinrichtung nach einem der Ansprüche 3 bis 7, **dadurch gekennzeichnet, dass** die Dichtlippe (51) in Abhängigkeit von der Schwenkrichtung einseitig druckverstärkend und druckvermindernd orientiert ausgebildet ist.

9. Die technische Gelenkeinrichtung nach einem der Ansprüche 3 bis 8, **dadurch gekennzeichnet, dass** mehrere Dichtlippen (51) oder Kontaktbereiche (52) hintereinander angeordnet sind, die kaskadiert druckaktivierbar ausgebildet sind.

10. Orthopädietechnische Gelenkeinrichtung nach einem der voranstehenden Ansprüche, **dadurch gekennzeichnet, dass** die Aufnahme (42) umlaufend an den Seitenkanten und der Kopfseite des Schwenkkolbens (40) oder als Zapfen ausgebildet ist.

11. Orthopädietechnische Gelenkeinrichtung nach einem der voranstehenden Ansprüche, **dadurch gekennzeichnet, dass** die Aufnahme (42) in einem Drehzapfen (45) ausgebildet ist, an dem der Schwenkkolben (40) angeordnet oder angeformt ist.

12. Orthopädietechnische Gelenkeinrichtung nach einem der voranstehenden Ansprüche, **dadurch gekennzeichnet, dass** der Schwenkkolben (40) mehrteilig ausgebildet ist und zwischen zwei Schwenkkolbenteilen (46, 47) die Aufnahme (42) ausgebildet ist.

13. Orthopädietechnische Gelenkeinrichtung nach einem der voranstehenden Ansprüche, **dadurch gekennzeichnet, dass** an dem Schwenkkolben (40) in oder an der Aufnahme (42) zumindest ein Formschlusselement (48) in einer Aufnahmewand (43) oder in einem Aufnahmegrund (44) und an der Dichtung (50) ein korrespondierend geformtes Formschlusselement (58) ausgebildet ist.

14. Orthopädietechnische Gelenkeinrichtung nach einem der voranstehenden Ansprüche, **dadurch gekennzeichnet, dass** es als künstliches Kniegelenk, Ellenbogengelenk oder Knöchelgelenk ausgebildet ist.

15. Orthopädietechnische Gelenkeinrichtung nach einem der voranstehenden Ansprüche, **dadurch gekennzeichnet, dass** in dem Kanal (35) zumindest eine Drossel (60) oder ein Ventil (61) angeordnet ist.

16. Orthopädietechnische Gelenkeinrichtung nach einem der voranstehenden Ansprüche, **dadurch gekennzeichnet, dass** die Dichtung (50) eine reibungsreduzierende Beschichtung oder reibungsreduzierende Oberflächenbehandlung aufweist.

## Claims

1. An orthopedic joint device having an upper part (10) and a lower part (20), which are mounted on one another so that they can pivot about a common pivot axis (15), and having rotary hydraulics (30) which comprise a hydraulic fluid, and which comprise a housing (31) with a chamber (32) and, mounted pivotably therein, a pivoting piston (40) which subdivides the chamber (32) into a flexion chamber (33) and an extension chamber (34) that are hydraulically connected to one another via at least one channel (35), wherein the pivoting piston (40) comprises a seat (42) in or on which a seal (50) that acts in the direction of the housing (31) is arranged, **characterized in that** the seal (50) is configured to experience a force acting away from the housing (31) due to the hydraulic fluid in a first pivoting direction and to experience a force acting toward the housing (31) in the opposite pivoting direction.

2. The orthopedic joint device as claimed in claim 1, **characterized in that** the seal (50) is prestressed, in particular elastically prestressed, in the direction of the housing (31).

3. The orthopedic joint device as claimed in claim 1 or 2, **characterized in that** the seal (50) comprises at least one sealing lip (51) on which at least one contact region (52) for bearing on the housing (31) is formed, and the sealing lip (51) has an inclined contour.

4. The orthopedic joint device as claimed in claim 3, **characterized in that** the sealing lip (51) is followed by a radial portion (53), which extends inward from the sealing lip (51) and is supported on a seat wall (43) or on a carrier (70).

5. The orthopedic joint device as claimed in claim 4, **characterized in that** the seal (50) comprises a base portion (54), which follows on from the radial portion (53) and is supported on the seat bottom (44) or on the carrier (70).

6. The orthopedic joint device as claimed in claim 5, **characterized in that** a reservoir (55) for hydraulic fluid is formed between the base portion (54) and the sealing lip (51).

7. The orthopedic joint device as claimed in one of claims 3 to 6, **characterized in that** the sealing lip (51) is configured to be inclined in the direction of the flexion chamber (33).

8. The orthopedic joint device as claimed in one of claims 3 to 7, **characterized in that** the sealing lip (51) is configured to be oriented so that it reinforces pressure and reduces pressure on one side as a function of the pivoting direction.

9. The orthopedic joint device as claimed in one of claims 3 to 8, **characterized in that** a plurality of sealing lips (51) or contact regions (52), which are configured to be pressure-activatable in cascade, are arranged in succession.

10. The orthopedic joint device as claimed in one of the preceding claims, **characterized in that** the seat (42) is formed circumferentially on the side edges and on the head side of the pivoting piston (40) or as a journal.

11. The orthopedic joint device as claimed in one of the preceding claims, **characterized in that** the seat (42) is formed in a rotating journal (45), on which the pivoting piston (40) is arranged or molded.

12. The orthopedic joint device as claimed in one of the preceding claims, **characterized in that** the pivoting piston (40) is configured in multiple parts and the seat (42) is formed between two pivoting piston parts (46, 47).

13. The orthopedic joint device as claimed in one of the preceding claims, **characterized in that** on the pivoting piston (40), in or on the seat (42), at least one form-fit element (48) is formed in a seat wall (43) or in a seat bottom (44) and a correspondingly shaped form-fit element (58) is formed on the seal (50).

14. The orthopedic joint device as claimed in one of the preceding claims, **characterized in that** it is configured as an artificial knee joint, elbow joint or ankle joint.

15. The orthopedic joint device as claimed in one of the preceding claims, **characterized in that** at least one throttle (60) or valve (61) is arranged in the channel (35).

16. The orthopedic joint device as claimed in one of the preceding claims, **characterized in that** the seal (50) comprises a friction-reducing coating or friction-reducing surface treatment.

## Revendications

1. Dispositif d'articulation orthopédique comprenant une partie supérieure (10) et une partie inférieure (20) qui sont montées l'une sur l'autre de manière à pouvoir pivoter autour d'un axe d'articulation commun (15), et un système hydraulique de rotation (30) qui comprend un fluide hydraulique et un boîtier (31) muni d'une chambre (32) et d'un piston pivotant (40) monté dans celle-ci de manière à pouvoir pivoter, lequel subdivise la chambre (32) en une chambre de flexion (33) et en une chambre d'extension (34) qui sont reliées hydrauliquement entre elles par au moins un canal (35), le piston pivotant (40) présentant un logement (42) dans ou sur lequel est disposé un joint d'étanchéité (50) agissant en direction du boîtier (31),
**caractérisé en ce que** le joint d'étanchéité (50) est conçu pour être soumis, dans un premier sens de pivotement, à une force agissant en éloignement du boîtier (31) et, dans le sens de pivotement opposé, à une force agissant vers le boîtier (31), sous l'effet du fluide hydraulique.

2. Dispositif d'articulation orthopédique selon la revendication 1,
**caractérisé en ce que** le joint d'étanchéité (50) est précontraint en direction du boîtier (31), en particulier précontraint de manière élastique.

3. Dispositif d'articulation orthopédique selon la revendication 1 ou 2,
**caractérisé en ce que** le joint d'étanchéité (50) présente au moins une lèvre d'étanchéité (51) sur laquelle est réalisée au moins une zone de contact (52) destinée à venir en appui contre le boîtier (31), ladite lèvre d'étanchéité (51) présentant un contour incliné.

4. Dispositif d'articulation orthopédique selon la revendication 3,
**caractérisé en ce qu'**une portion radiale (53) se raccorde à la lèvre d'étanchéité (51), laquelle s'étend vers l'intérieur depuis la lèvre d'étanchéité (51) et s'appuie contre une paroi de logement (43) ou un support (70).

5. Dispositif d'articulation orthopédique selon la revendication 4,
**caractérisé en ce que** le joint d'étanchéité (50) présente une portion de base (54) qui se raccorde à la portion radiale (53) et s'appuie sur le fond de logement (44) ou le support (70).

6. Dispositif d'articulation orthopédique selon la revendication 5,
**caractérisé en ce qu'**un espace de stockage (55) pour le fluide hydraulique est ménagé entre la portion de base (54) et la lèvre d'étanchéité (51).

7. Dispositif d'articulation orthopédique selon l'une des revendications 3 à 6, **caractérisé en ce que** la lèvre d'étanchéité (51) est inclinée en direction de la chambre de flexion (33).

8. Dispositif d'articulation orthopédique selon l'une des revendications 3 à 7, **caractérisé en ce que** la lèvre d'étanchéité (51) est orientée de manière à amplifier ou à réduire la pression d'un côté en fonction du sens de pivotement.

9. Dispositif d'articulation orthopédique selon l'une des revendications 3 à 8, **caractérisé en ce que** plusieurs lèvres d'étanchéité (51) ou zones de contact (52) sont disposées les unes derrière les autres et sont conçues pour être activées en cascade par pression.

10. Dispositif d'articulation orthopédique selon l'une des revendications précédentes,
**caractérisé en ce que** le logement (42) est réalisé circonférentiellement sur les bords latéraux et du côté tête du piston pivotant (40) ou sous forme de tenon.

11. Dispositif d'articulation orthopédique selon l'une des revendications précédentes,
**caractérisé en ce que** le logement (42) est réalisé dans un pivot (45) sur lequel le piston pivotant (40) est disposé ou formé.

12. Dispositif d'articulation orthopédique selon l'une des revendications précédentes,
**caractérisé en ce que** le piston pivotant (40) est réalisé en plusieurs parties, et le logement (42) est réalisé entre deux parties de piston pivotant (46, 47).

13. Dispositif d'articulation orthopédique selon l'une des revendications précédentes,
**caractérisé en ce qu'**au moins un élément de liaison par complémentarité de forme (48) est réalisé dans ou sur le logement (42) du piston pivotant (40), dans une paroi de logement (43) ou dans un fond de logement (44), et un élément de liaison par complémentarité de forme (58) de forme correspondante est réalisé sur le joint d'étanchéité (50).

14. Dispositif d'articulation orthopédique selon l'une des revendications précédentes,
**caractérisé en ce qu'**il est conçu comme une articulation artificielle du genou, du coude ou de la cheville.

15. Dispositif d'articulation orthopédique selon l'une des revendications précédentes,
**caractérisé en ce qu'**au moins un étranglement (60) ou une soupape (61) est disposé(e) dans le canal (35).

16. Dispositif d'articulation orthopédique selon l'une des revendications précédentes,
**caractérisé en ce que** le joint d'étanchéité (50) présente un revêtement réduisant le frottement ou un traitement de surface réduisant le frottement.
